# FASCICULE DE BREVET EUROPEEN

(11) **EP 1 409 444 B1**
(45) Date de publication et mention de la délivrance du brevet: **02.11.2006**
(21) Numéro de dépôt: 02762492.3
(22) Date de dépôt: 26.06.2002
(51) Int. Cl.: C07C 51/12, C07C 67/37, C07C 53/08, C07C 69/14

(54) **PERFECTIONNEMENT AUX PROCEDES DE FABRICATION D'ACIDE ACETIQUE ET/OU D'ACETATE DE METHYLE EN CONTINU**
VERBESSERTE VERFAHREN ZUR KONTINUIERLICHEN HERSTELLUNG VON ESSIGSÄURE UND/ODER ESSIGSÄUREMETHYLESTER
IMPROVEMENT TO METHODS FOR THE CONTINUOUS PRODUCTION OF ACETIC ACID AND/OR METHYL ACETATE

(30) Priorité: 03.07.2001 FR 0108798
(43) Date de publication de la demande: 21.04.2004
(73) Titulaire: ACETEX CHIMIE, 92205 Neuilly-sur-Seine (FR)
(72) Inventeur: Thiebaut, Daniel, 94400 Courbevoie (FR)
(74) Mandataire: Giraud, Françoise
(86) Numéro de dépôt international: PCT/FR2002/002212
(87) Numéro de publication internationale: WO 2003/004449

(56) Documents cités:
- EP-A- 0 161 874
- EP-A- 1 002 785
- WO-A-02/04394
- US-A- 5 352 415
- US-A- 5 374 774
- US-A- 5 604 132

## Description

L'invention concerne un perfectionnement aux procédés de fabrication d'acide acétique et/ou d'acétate de méthyle en continu.

Plus précisément, ce perfectionnement vise à améliorer le contrôle et la conduite d'un procédé de préparation d'acide acétique et/ou d'acétate de méthyle, en vue d'améliorer la production d'acide acétique et/ou d'acétate de méthyle.

Parmi les procédés couramment utilisés pour fabriquer l'acide acétique, l'un des plus utilisés industriellement est la carbonylation du méthanol ou plus généralement d'un dérivé carbonylable de méthanol par le monoxyde de carbone. Cette réaction est mise en oeuvre en phase liquide, sous pression de monoxyde de carbone, qui est l'un des réactifs, en présence d'un système catalytique homogène.

Le procédé de carbonylation au rhodium est un procédé connu, exploité industriellement et ayant fait l'objet de nombreux articles et brevets comme par exemple, les brevets américains US 3,769,329 et US 3,813,428.

Les brevets européens EP 618 183 et EP 618 184 ainsi que les brevets européens EP 785 919 et EP 759 022 décrivent un procédé de carbonylation du méthanol, en présence d'un système catalytique à base d'iridium, et le cas échéant, contenant en plus du rhodium.

Un procédé de carbonylation à l'iridium et au ruthénium, actuellement exploité industriellement, est décrit dans le brevet européen EP 643 034.

L'amélioration de ces procédés de carbonylation du méthanol a visé à augmenter la productivité des catalyseurs et à diminuer les coûts de fabrication de l'acide acétique.

Les procédés dits "à basse teneur en eau" permettent en effet d'augmenter fortement la production d'acide acétique, en limitant le niveau d'investissement requis et en abaissant les coûts opératoires par diminution de l'énergie de séparation de l'acide acétique des différents constituants du mélange réactionnel, en particulier l'eau.

De façon classique, ces différents procédés de carbonylation du méthanol, en phase liquide, et en présence d'un système catalytique homogène sont mis en oeuvre dans des installations comprenant trois zones distinctes, comme décrit dans l'article de M.J. HOWARD et al, Catalysis Today, 18, (1993) 325-354.

La zone de réaction est constituée d'un réacteur continu agité opérant sous pression (5-200 bars) et à température élevée (150-250°C).

Le méthanol alimentaire et un certain nombre de courants de recyclage sont introduits à la base du réacteur. Le monoxyde de carbone est dispersé dans le réacteur.

Le milieu réactionnel liquide produit est ensuite envoyé dans la deuxième zone dite zone de vaporisation ou zone de flash où le liquide est partiellement vaporisé à une pression inférieure à celle de réaction.

Cela crée un flash ou détente adiabatique où la majorité des constituants légers (iodure de méthyle, acétate de méthyle et eau) ainsi que l'acide produit sont vaporisés.

La zone de flash permet la séparation du gaz du liquide, le flux vaporisé passe ensuite dans la troisième zone dite de séparation, alors que le flux liquide (essentiellement de l'acide acétique contenant le catalyseur) est recyclé vers la première zone.

La zone de purification peut comprendre une à plusieurs colonnes de distillation ; elle permet la séparation de l'acide acétique et/ou de l'acétate de méthyle des autres constituants et le recyclage des flux vers la zone de réaction.

En outre, une purge des gaz de la tête du réacteur permet de contrôler le niveau de pression partielle de monoxyde de carbone et d'éliminer les sous produits gazeux de la réaction, ainsi que les inertes présents dans le monoxyde de carbone alimentaire.

L'optimisation des procédés actuels de préparation d'acide acétique conduit à maximiser la production d'acide acétique, dans des équipements déjà existants. Pour cela, la réaction est conduite en maintenant les concentrations des différents constituants du milieu réactionnel à des valeurs prédéterminées pour bénéficier de conditions cinétiques les plus appropriées.

Le contrôle du réacteur et des flux recyclés devient alors primordial.

Différents articles et brevets récents commencent à couvrir ce domaine.

La présentation de D.Z. TOBIAS lors de la conférence IEEE, Advanced Process Control, VANCOUVER, 29-30 avril 1999 : "Adaptive Process Control of an Acetic Acid Reactor", montre l'utilisation d'un échangeur externe pour découpler bilan matière et bilan thermique, puisque sans cet échangeur, on ne peut faire varier le débit de sortie du réacteur pour maintenir la température dudit réacteur : le débit de sortie du réacteur est en effet imposé par le bilan matière.

L'objectif recherché est de stabiliser la température du réacteur, pour accroître la production de l'unité : la mise en place d'un nouveau contrôle a ainsi permis de réduire la déviation standard pour la température de 3.6 à 0.8°C.

Dans les opérations continues, il est habituel d'alimenter le monoxyde de carbone à la demande, sous le contrôle de la pression totale du réacteur.

La demande de brevet européenne EP 0 983 752 a pour objet la mise en place d'un système de contrôle qui vise à maintenir le débit de monoxyde de carbone en dessous d'un débit calculé maximum qui représente un débit maximum acceptable.

La demande de brevet européenne EP 0 999 198 vise à maintenir la composition du milieu réactionnel, en particulier la concentration en eau et en acétate de méthyle dans le réacteur de carbonylation en recyclant un flux d'acide acétique riche venant de la zone de purification de l'acide.

Les brevets EP 0 846 674 et FR 2 750 984 décrivent l'optimisation de la consommation de CO par l'introduction d'un deuxième réacteur de carbonylation (effet finisseur) entre le 1^{er} réacteur (zone de réaction) et le flash (zone de vaporisation).

Les brevets US 5 352 415 et US 5 374 774 décrivent des procédés de fabrication d'acide acétique dans lesquels on contrôle les niveaux du réacteur et du flash et la concentration en eau du milieu réactionnel.

Le brevet US 5 831 120 fournit une solution technique pour éviter l'accumulation d'eau dans le milieu réactionnel et obtenir une concentration en eau visée dans le réacteur de carbonylation.

Ces différents documents enseignent que, dans le procédé conventionnel développé par la société MONSANTO, la chaleur de réaction était soustraite par l'intermédiaire de la détente du débit de sortie du réacteur ou débit de flash allant du réacteur au flash. Ainsi, pour contrôler la température du réacteur, il y avait une relation fixe entre le débit de méthanol entrant dans le réacteur et le débit de flash. Cela conduisait à une faible variabilité du niveau du liquide dans le réacteur et des flux de process intermédiaires.

Avec l'introduction des procédés à basse teneur en eau, ce système devient inadéquat parce que le débit vaporisé au flash augmente fortement puisque les quantités d'eau évaporées sont plus faibles et qu'il faut les remplacer par des quantités beaucoup plus importantes de produits organiques, avec une chaleur latente de vaporisation plus faible que celle de l'eau.

On a donc été amené à mettre en place des échangeurs refroidisseurs pour absorber une partie de la chaleur de réaction mais on a augmenté alors la variabilité des niveaux liquides du réacteur et du flash, ainsi que des débits liquides.

Cette grande dispersion des débits liquides, en particulier, conduit à des déficiences de procédé, puisqu'on peut être amené à diminuer la production pour réduire le flux entrant dans la zone de purification.

De plus, ces fluctuations entraînent des variations de la concentration en eau dans le réacteur et, comme la cinétique de réaction dépend de la concentration en eau pour les procédés basse teneur en eau, cela augmente encore les risques d'instabilité du système.

La demande de brevet EP 1 002 785 vise à maintenir la concentration en acétate de méthyle dans le réacteur à une valeur prédéterminée en jouant sur le ratio entre le méthanol et le monoxyde de carbone, rapport qui régule le débit d'alimentation du méthanol au réacteur.

La demande internationale de brevet WO 00/37405 fournit une méthode de contrôle du procédé en mesurant les concentrations des différents composants de la solution réactionnelle au moyen d'un analyseur infra-rouge et en ajustant en réponse, les concentrations d'au moins l'espèce catalytique, l'iodure de méthyle, l'acétate de méthyle et l'eau, pour optimiser la fabrication de l'acide acétique.

En tout état de cause, le problème du contrôle du réacteur d'acide acétique et du maintien des concentrations notamment de l'eau et de l'acétate de méthyle, a bien été défini, mais rien ne suggère un moyen pour assurer ces objectifs de façon automatique.

Par ailleurs, aucun document ne se préoccupe des problèmes posés par les fluctuations plus ou moins importantes du flux de monoxyde de carbone entrant dans le réacteur et ne cherche à pallier les inconvénients qui peuvent en résulter sur la production d'acide acétique.

La présente invention a justement pour but de pallier ces inconvénients et fournit une méthode de contrôle qui agit de façon automatique pour maintenir les concentrations notamment en eau et/ou en acétate de méthyle et qui permet de maximiser la production d'acide acétique lorsque le débit d'introduction de CO subit des fluctuations, comme cela va être décrit.

On rappellera tout d'abord que :
La réaction principale de carbonylation du méthanol s'écrit :

   CO + CH₃OH → CH₃COOH
Les principales réactions secondaires sont :
   - La réaction de gaz à l'eau
      CO + H₂O → CO₂ + H₂ encore désigné par WGSR (Water Gas Shift Reaction)

Cette réaction entraîne une perte de monoxyde de carbone et une production simultanée d'hydrogène et de gaz carbonique.

Il est important d'évaluer quantitativement cette réaction secondaire par rapport à la réaction principale. Pour utiliser un critère indépendant du niveau de production, on parle de sélectivités hydrogène ou CO₂ en exprimant la sélectivité comme le rapport du nombre de moles de monoxyde de carbone ayant participé à la réaction secondaire, par rapport au nombre total de moles de monoxyde de carbone ayant participé à la réaction principale et aux réactions secondaires.
- La réaction de formation de l'acide propionique qui s'écrit globalement :

   CH₃COOH + 2 H₂ + CO → H₂O + CH₃CH₂COOH

   De l'hydrogène et du monoxyde de carbone sont ainsi consommés.
   On peut ici remarquer qu'une partie de l'hydrogène généré par la réaction précédente de gaz à l'eau est consommée par la réaction de sous-production d'acide propionique. En analysant le flux de gaz de sortie du réacteur, on n' a plus alors équivalence entre sélectivité hydrogène et sélectivité CO₂.
   La sélectivité CO₂ est plus représentative de la réaction de gaz à l'eau, mais, il n'est pas exclu, pour des raisons pratiques liées à l'analyse des gaz, d'utiliser la sélectivité hydrogène pour conduire l'installation industrielle.
- La réaction d'estérification de l'acide acétique et du méthanol en acétate de méthyle ;

L'acétate de méthyle ainsi formé peut gêner la séparation du flux liquide condensé en tête de la première colonne de purification, si sa concentration augmente de façon incontrôlée dans le réacteur.

Selon le procédé de production de monoxyde de carbone en amont du réacteur d'acide acétique, le flux de monoxyde de carbone ne peut être totalement constant. De petites variations peuvent être amorties en plaçant en amont du réacteur d'acide acétique, un réservoir tampon de monoxyde de carbone, qui permet d'atténuer et de lisser dans le temps les variations du débit de monoxyde de carbone, dans la mesure où le niveau de pression de la source de monoxyde de carbone est supérieur au niveau de la pression de l'utilisation dans le réacteur de carbonylation.

Les fluctuations plus importantes conduisent à ne pouvoir utiliser dans la réaction un flux de monoxyde de carbone que l'on peut définir par CO en excès qui doit alors être rejeté à l'atmosphère soit par l'intermédiaire d'une torche, soit par un système de combustion avec récupération de chaleur.

Le perfectionnement selon l'invention consiste à faire varier la température du réacteur et le débit d'entrée du méthanol dans le réacteur dans le but d'ajuster la production d'acide acétique au débit de monoxyde de carbone disponible, tout en maintenant une sélectivité hydrogène ou CO₂ basse, ou des conditions permettant d'optimiser la consommation du CO et cela, notamment en recourant à un dispositif électronique préalablement programmé tel qu'un régulateur multivariable à commande prédictive.

Ainsi, contrairement aux modes opératoires précédents où la température du réacteur restait fixée à une valeur déterminée pendant de longues périodes, selon l'invention, la température du réacteur varie pour pouvoir utiliser tout le monoxyde de carbone disponible.

Il est apparu que, même si les paramètres utilisés comme variables d'entrée dans le régulateur n'étaient pas les mêmes selon qu'il s'agissait d'un procédé fonctionnant selon le mode conventionnel avec une teneur en eau du milieu réactionnel supérieure ou égale à 14 % ou d'un procédé dit "basse teneur en eau", il était dans tous les cas possible d'optimiser la production d'acide acétique et/ou d'acétate de méthyle en recourant à un régulateur agissant sur la température du réacteur et le débit d'entrée du méthanol (variables de sortie ou variables d'action), en fonction du débit d'entrée du monoxyde de carbone et d'au moins un paramètre supplémentaire choisi comme consigne (valeurs d'entrée ou valeurs objectifs).

Ainsi, selon sa caractéristique essentielle, l'invention concerne un procédé de préparation en continu d'acide acétique et/ou d'acétate de méthyle par carbonylation par du monoxyde de carbone, du méthanol, ou d'un dérivé carbonylable du méthanol, en phase liquide, en présence d'eau et d'un système catalytique homogène, ledit procédé de préparation étant mis en oeuvre dans une installation industrielle comprenant :
- Une zone I dite zone de réaction, comprenant un réacteur dans lequel est réalisée ladite réaction de carbonylation du méthanol, en phase liquide, à une température de 150 à 250 °C, sous une pression de 5.10⁵ à 200.10⁵ Pa et avec élimination sous forme d'évents d'une partie du ciel gazeux au dessus du niveau liquide du milieu réactionnel dans ledit réacteur,
- Une zone II dite zone de vaporisation ou zone de flash où le liquide issu du milieu réactionnel de la zone I est partiellement vaporisé à une pression inférieure à celle de la zone I, la fraction liquide issue de cette vaporisation partielle étant recyclée vers le réacteur,
- Une zone III dite zone de purification par distillation de la fraction vaporisée issue dudit flash de la zone II, sur une ou plusieurs colonnes de distillation, à la sortie de laquelle/desquelles on récupère l'acide acétique et/ou l'acétate de méthyle, les autres constituants de ladite fraction vaporisée étant au moins partiellement recyclés vers ledit réacteur,
caractérisé en ce que, en vue d'améliorer le contrôle de la production d'acide acétique et/ou d'acétate de méthyle, on asservit la température du réacteur et le débit d'entrée du méthanol ou du dérivé carbonylable dans ledit réacteur au débit d'entrée de monoxyde de carbone et à au moins un des paramètres définissant la composition du milieu réactionnel et/ou des évents.

La présente invention fournit donc un moyen pour mieux contrôler la production d'acide acétique et/ou d'acétate de méthyle dans un procédé en continu de carbonylation du méthanol ou d'un dérivé carbonylable du méthanol par du monoxyde de carbone, en phase liquide, en présence d'eau et d'un système catalytique homogène mis en oeuvre dans une installation industrielle comprenant trois zones principales I, II et III définies ci-dessus.

Par dérivé carbonylable du méthanol, on entend tous les dérivés du méthanol classiquement utilisés dans les procédés industriels de préparation de l'acide acétique et/ou de l'acétate de méthyle par carbonylation, par exemple, le diméthyléther, les halogénures de méthyle ou l'acétate de méthyle.

Le contrôle effectué dans le cadre du procédé selon l'invention peut être mis en oeuvre au moyen de tout dispositif électronique permettant d'assurer l'asservissement recherché, de façon à minimiser les pertes en monoxyde de carbone lorsque l'alimentation en monoxyde de carbone subit des fluctuations.

Ce dispositif d'asservissement peut, en particulier, être un dispositif électronique de régulation préalablement programmé à cet effet.

Plus précisément, le dispositif d'asservissement agit sur la température du réacteur et sur le débit d'entrée du méthanol dans le réacteur, de façon à minimiser les pertes en monoxyde de carbone et, cela, en contrôlant, de façon continue, le débit d'entrée du monoxyde de carbone dans le réacteur et au moins un des paramètres définissant la composition du milieu réactionnel et/ou des évents.

L'homme du métier comprendra aisément que le paramètre choisi, comme entrée du dispositif destiné à réaliser l'asservissement, dépend du mode de fonctionnement du procédé de fabrication de l'acide acétique et/ou de l'acétate de méthyle et, qu'en particulier, ces paramètres pourront être différents, suivant qu'il s'agit d'un procédé dit conventionnel de type Monsanto, ou d'un procédé dit "basse teneur en eau", comme cela apparaît dans la description qui suit.

Le dispositif de régulation peut être soit un système de contrôle-commande s'il en présente les fonctionnalités requises, soit un régulateur multivariable prédictif ou tout autre système électronique présentant les caractéristiques suivantes:
- Un dispositif d'entrées/sorties (I/O)
- Une interface de conversion numérique des entrées/sorties analogiques
- Un processeur de calcul.

Toutefois on a choisi, au titre des exemples, un régulateur multivariable prédictif pour s'affranchir des particularismes liés aux systèmes de contrôle-commande d'une part, et par ailleurs pour pouvoir directement utiliser les programmes fournis avec le régulateur multivariable.

Un tel dispositif est basé sur l'utilisation d'un modèle mathématique de régulation et permet une commande prédictive qui, en s'appuyant sur ce modèle mathématique, réalise une hypothèse sur le futur de la variable à contrôler.

Les régulateurs multivariables prédictifs disponibles dans le commerce incorporent généralement une bibliothèque de modèles mathématiques de régulation.

Avant toute utilisation, un régulateur multivariable prédictif doit être programmé. La programmation d'un régulateur multivariable prédictif est généralement effectuée de la façon suivante :
- On choisit, dans la bibliothèque précitée, un modèle mathématique en fonction du réacteur utilisé et de la réaction chimique que l'on souhaite mettre en oeuvre. Ce choix est typiquement fait de façon empirique, au moyen d'essais préalables consistant à tester tous les modèles mathématiques sur la réaction chimique considérée et à observer les réponses de régulation obtenues.
- On fournit ensuite au régulateur des valeurs de variables dites d'action et des valeurs correspondantes de variables dites objectifs. Les variables d'action sont les variables sur lesquelles on va agir pour que les variables objectifs soient régulées autour de valeurs de consigne souhaitées. Dans la présente invention, les variables d'action sont constituées par la température du réacteur et le débit de méthanol, et les variables objectifs sont constituées par le débit d'entrée de monoxyde de carbone et le paramètre susmentionné définissant la composition du milieu réactionnel et/ou des évents.

Cette seconde phase revient à créer, dans le régulateur, une base de données représentant les relations entre les variables d'action et les variables objectifs.

Un programme de calcul, fourni avec le régulateur, optimise ensuite les paramètres de régulation, tels que le gain et le retard, qui devront être appliqués aux signaux électriques de sortie du régulateur pour commander des organes (typiquement des vannes) agissant sur les variables d'action.

Les valeurs des variables d'action et des variables objectifs qui sont fournies au régulateur, sont obtenues au cours d'une phase expérimentale préalable, sans régulation, consistant à mettre en oeuvre la réaction chimique, à incrémenter rapidement la valeur de l'une des variables afin de faire évoluer le système, et à observer l'évolution des différentes variables en mesurant de façon continue leur valeur.

Une fois le régulateur programmé, celui-ci est intégré dans le poste de conduite et est progressivement bouclé sur le procédé.

Lors de la mise en oeuvre du procédé, le régulateur régule les variables objectifs autour de valeurs de consigne, en agissant sur les variables d'action par l'intermédiaire des organes précités.

Selon une première variante, l'invention s'applique au contrôle des procédés conventionnels de carbonylation du méthanol en acide acétique et/ou en acétate de méthyle mettant en oeuvre des teneurs supérieures ou égales à 14 % en eau.

Il est bien connu que, dans un tel procédé conventionnel de carbonylation du méthanol en acide acétique et/ou en acétate de méthyle, en phase liquide, catalysé par le rhodium, avec des teneurs en eau supérieures ou égales à 14 %, le monoxyde de carbone est introduit sous le contrôle de la pression totale du réacteur; le méthanol est introduit à débit fixé pour obtenir la production désirée d'acide acétique à température fixée du réacteur.

Ce mode de contrôle fonctionne si l'activité du catalyseur est suffisante.

Si ce n'est pas le cas, le méthanol non transformé en acide acétique, s'estérifie en acétate de méthyle. La montée de la concentration de l'acétate de méthyle dans le réacteur engendre de mauvais fonctionnements dans la zone de purification, en particulier, en tête de la première colonne de purification où la montée de la concentration en acétate de méthyle détériore d'abord, puis inhibe la séparation du liquide condensé en deux phases liquides distinctes (une phase aqueuse légère qui d'une part sert au reflux de la colonne et d'autre part est recyclée au réacteur, et une phase organique lourde qui est intégralement recyclée au réacteur de carbonylation).

Le niveau de production doit alors être diminué, pour retrouver une situation correcte, pour ensuite ajouter du catalyseur supplémentaire ou augmenter la température du réacteur, pour permettre de travailler avec un niveau de production plus élevé.

On a trouvé qu'une meilleure conduite de la réaction pouvait être obtenue en contrôlant la réaction secondaire de gaz à l'eau qui produit de l'hydrogène et du gaz carbonique.

L'analyse en continu ou de façon séquentielle des gaz de purge de l'atelier, couplée à la mesure du débit total des évents de l'atelier permet de déterminer les débits d'hydrogène et de gaz carbonique générés par les réactions secondaires dans le réacteur de carbonylation.

On a ainsi accès aux sélectivités en rapportant ces débits partiels au débit total de monoxyde de carbone entrant dans le réacteur.

On peut ici remarquer qu'une partie de l'hydrogène généré par la réaction de gaz à l'eau WGSR est consommée par la réaction de sous-production d'acide propionique. On n' a plus alors équivalence entre sélectivité hydrogène et sélectivité CO₂.

La sélectivité CO₂ est plus représentative de la réaction de gaz à l'eau, mais, certaines fois pour des raisons pratiques, liées à l'analyse des gaz, on pourra utiliser la sélectivité hydrogène pour conduire l'installation industrielle.

Ainsi, selon la première variante de l'invention, un bon contrôle de la réaction est obtenu en faisant varier le débit de méthanol ou du dérivé carbonylable de méthanol utilisé à l'entrée du réacteur et la température du réacteur, pour obtenir une sélectivité en hydrogène ou CO₂ inférieure ou égale à 0.01 et/ou une concentration en acétate de méthyle dans le réacteur inférieure à 5 % en poids, de préférence inférieure à 2 % en poids, ce qui permet d'assurer une bonne décantation en tête de la première colonne de purification.

Les essais réalisés par l'inventeur de la présente invention, ont permis de mettre clairement en évidence, qu'il était possible, en agissant par l'intermédiaire du régulateur, sur la température du réacteur et sur le débit d'entrée de CO, de limiter les pertes en CO lorsque le débit d'entrée du monoxyde de carbone dans le réacteur varie, et cela, en imposant à la sélectivité en CO₂ ou H₂, une valeur de consigne inférieure ou égale à 0.01 et/ou en maintenant la concentration en acétate de méthyle à une valeur inférieure à 5 % en poids dans le milieu réactionnel et préférence inférieure à 2 %.

Ainsi, selon cette première variante où la concentration en eau dans le milieu réactionnel est supérieure ou égale à 14 % en poids, la régulation porte sur la sélectivité en CO₂ ou en H₂ et le débit de CO à consommer et le régulateur agit à la fois sur la température du réacteur et sur le débit de méthanol (ou de dérivé carbonylable) entrant dans le réacteur.

Le recours au dispositif régulateur dans cette première variante de l'invention, a permis de maintenir la sélectivité en CO₂ (ou en hydrogène) dans un faible domaine de variation, en jouant sur la température du réacteur et sur le débit d'entrée du méthanol (ou du dérivé carbonylable) dans le réacteur, et a permis d'optimiser la consommation du monoxyde de carbone, même en présence de fluctuations relativement importantes du débit d'entrée du monoxyde de carbone dans le réacteur.

Selon une seconde variante, l'invention s'applique également aux procédés de fabrication d'acide acétique et/ou d'acétate de méthyle en phase liquide en présence d'un catalyseur homogène, dit procédé "basse teneur en eau", c'est à dire au cas où la concentration en eau dans le milieu réactionnel est inférieure à 14 % en poids.

Il est bien connu que, dans de tels procédés "basse teneur en eau", contrairement aux procédés conventionnels où la concentration en eau est supérieure ou égale à 14 % en poids du milieu réactionnel, la concentration en eau est un paramètre direct de la cinétique de la réaction de production de l'acide acétique, alors que la réaction de carbonylation est relativement peu sensible à la sélectivité CO₂, c'est pourquoi l'homme du métier comprendra aisément que dans un tel cas, contrairement au cas précédent, on ne retiendra pas la sélectivité CO₂ comme un paramètre utilisé comme variable objectif du régulateur mais plutôt la concentration en eau dans le milieu réactionnel.

Ainsi donc, pour les procédés de fabrication "basse teneur en eau", on retiendra avantageusement comme variables objectifs du régulateur, la concentration en eau qui sera fixée à une valeur prédéterminée et le débit de monoxyde de carbone entrant dans le réacteur.

Il est apparu que, dans ce cas encore, il était possible d'agir par l'intermédiaire d'un régulateur préalablement programmé, en particulier par l'intermédiaire d'un régulateur multivariable prédictif, sur la température du réacteur et sur le débit de méthanol entrant dans le réacteur, de façon à maintenir la concentration en eau à ladite valeur prédéterminée et à optimiser la consommation de monoxyde de carbone lorsque l'alimentation en ce dernier subit des fluctuations.

Selon une variante avantageuse applicable aux deux modes de réalisation de l'invention précédemment exposés, il est apparu particulièrement utile d'asservir en outre, le débit du liquide issu de la zone I de réaction vers la zone II de flash et les débits de liquide de recyclage des zones II et III vers le réacteur au niveau du liquide dans le réacteur, de façon à ce que le niveau reste fixé à une valeur prédéterminée.

Cette valeur prédéterminée est avantageusement fixée à une valeur comprise entre 50 et 100 % de l'échelle totale absolue des niveaux dans le réacteur.

Ainsi, le régulateur utilisé selon l'invention peut également en outre être utilisé pour régler le niveau de liquide dans le réacteur et, cela en ajoutant comme variable d'action au régulateur le débit du liquide du réacteur vers le flash et les différents débits de retour vers le réacteur, en particulier les débits et flux liquides provenant des zones II et III.

Selon une autre variante particulièrement avantageuse de l'invention, le dispositif d'asservissement utilisé selon l'invention, en particulier le régulateur multivariable prédictif, peut être utilisé pour contrôler et réguler la teneur en eau dans le milieu réactionnel.

Il peut en particulier être utilisé pour contrôler et réguler d'une part les équipements permettant d'éviter l'accumulation d'eau dans le milieu réactionnel en particulier dans le cas d'une colonne à distiller permettant de soustraire de l'eau au procédé de préparation d'acide acétique.

Il peut également être utilisé, avec le même objectif de contrôler la teneur en eau dans le milieu réactionnel, pour contrôler et réguler le ou lesdits débits d'acétate de méthyle, de diméthyléther, d'anhydride acétique introduit en remplacement d'une partie du méthanol alimentaire, et cela dans le but d'ajuster la teneur en eau dans le réacteur.

Selon une autre variante particulièrement avantageuse de l'invention et, de manière à soulager le train de purification, on peut ajouter des échangeurs permettant d'absorber une partie des calories de la réaction de production d'acide acétique. Le contrôle de la température du réacteur repose alors sur le contrôle des calories ainsi échangées et on a pu montrer que le système selon l'invention permettait de bien pouvoir utiliser efficacement tout le monoxyde de carbone disponible avec une bonne maîtrise de la composition du réacteur (acétate de méthyle).

Il est possible, selon une autre variante de l'invention, d'éliminer ou de récupérer une partie des calories de réaction de production d'acide acétique.

Cette élimination ou cette récupération peut être réalisée soit à la sortie du réacteur par l'intermédiaire d'un échangeur de chaleur placé sur une boucle de recirculation de liquide réactionnel vers ledit réacteur ou à l'entrée du réacteur sur des flux de recyclage vers ledit réacteur.

Selon une autre variante, on peut amortir les fluctuations, les variations du débit d'entrée du monoxyde de carbone par l'intermédiaire d'un réservoir tampon placé en amont dudit réacteur.

Selon cette variante, on fait dépendre une valeur de consigne du débit de monoxyde de carbone de la pression à l'intérieur dudit réservoir tampon.

Selon une autre variante de l'invention, il est possible d'amortir les fluctuations de débit de monoxyde de carbone entrant dans le réacteur en rejetant au moins une partie du débit excédentaire vers l'atmosphère.

Ce rejet pourra être réalisé, en particulier, de façon classique, soit par l'intermédiaire d'une torche, soit par l'intermédiaire d'un système de récupération de chaleur.

Enfin, il est apparu qu'il était particulièrement avantageux de coupler le dispositif de régulation utilisé selon l'invention avec un analyseur opérant dans le domaine du proche infra-rouge pour mesurer les concentrations en eau et en acétate de méthyle dans le milieu réactionnel.

Ce même type de couplage pourra également être réalisé pour mesurer les concentrations en eau et en acétate de méthyle et/ou en iodure de méthyle dans le milieu réactionnel lorsque l'iodure de méthyle sera utilisé comme co-catalyseur selon une variante avantageuse du procédé selon l'invention.

Ce couplage d'un analyseur de procédé temps réel basé sur une analyse dans le domaine du proche infra-rouge, s'est avéré particulièrement intéressant dans les procédés à "basse teneur en eau" où il importe de maîtriser, outre la concentration en acétate de méthyle, la teneur en eau qui influe directement sur la cinétique de la réaction de production d'acide acétique et/ou d'acétate de méthyle.

D'une façon générale, le perfectionnement selon l'invention s'applique à tous les procédés de fabrication d'acide acétique et/ou d'acétate de méthyle en phase liquide en continu, en présence d'un système catalytique homogène.

Il s'applique tout particulièrement aux procédés de fabrication dont le système catalytique comprend au moins un métal du groupe VIII, en particulier du rhodium, de l'iridium ou du platine.

Il s'applique également, de façon particulièrement avantageuse, aux procédés de carbonylation dans lesquels le système catalytique comprend, en outre, au moins un co-catalyseur, et en particulier de l'iodure de méthyle.

L'invention apparaîtra plus clairement au vu des exemples qui suivent, décrits en référence aux figures annexées qui représentent respectivement :
- Figure 1 : le schéma de l'installation de fabrication d'acide acétique par carbonylation selon l'art antérieur. Cette figure est donnée en référence à l'exemple 1.
- Figure 2 : le schéma d'une installation perfectionnée selon l'invention. Cette figure est donnée en référence à l'exemple 2.

### EXEMPLES

### Exemple 1 (comparatif)

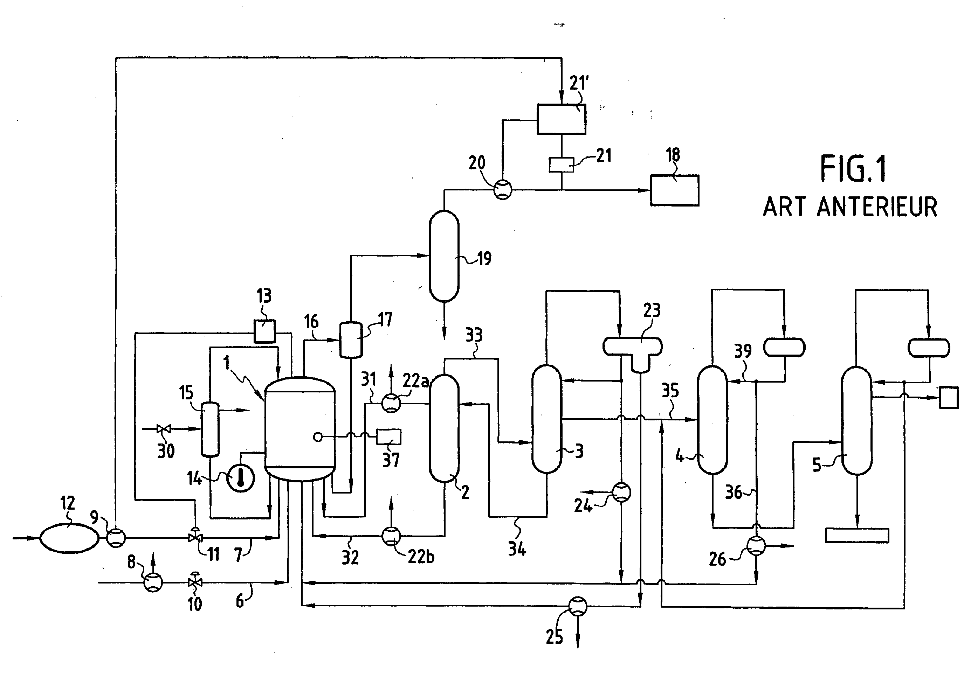

On prépare dans cet exemple de l'acide acétique par carbonylation du méthanol par le monoxyde de carbone, en phase liquide, en présence d'un système catalytique homogène, dans une installation représentée schématiquement sur la figure 1.

La réaction de carbonylation est réalisée à une température de 185°C sous une pression totale de 29 bar absolus.

La concentration en eau est maintenue à une valeur de 14% en poids dans le milieu réactionnel.

Les concentrations exprimées en poids du milieu réactionnel sont les suivantes :
- Eau : 14%
- Iodure de méthyle : 10%
- Acétate de méthyle : 1,75%
- Acide acétique et catalyseurs : le complément à 100%.

La production d'acide acétique est de 54,1 T/h.

L'installation comprend une zone de réaction I constituée essentiellement d'un réacteur 1, une zone II de flash comprenant essentiellement un dispositif de flash 2 et une zone III de purification comprenant, dans le cas représenté sur cette figure, trois colonnes de distillation 3, 4 et 5.

Le monoxyde de carbone est fourni après purification dans un système cryogénique (non représenté sur la figure) de séparation par lavage au méthane liquide.

Sa pureté varie de 98 à 99 % (ces pourcentages sont exprimés en volume/volume) selon le fonctionnement des équipements de purification.

Le monoxyde de carbone est ensuite introduit au fond du réacteur 1 par l'intermédiaire d'une canalisation 7 munie d'un débitmètre 9 et d'une vanne d'entrée 11. Un réservoir tampon de monoxyde de carbone 12 placé en amont de la vanne d'entrée 11 permet, le cas échéant, d'amortir des variations dans le débit d'entrée de CO dans le réacteur 1.

Le méthanol est introduit également au fond du réacteur 1 par l'intermédiaire d'une canalisation 6 munie d'un débitmètre 8 et d'une vanne d'entrée 10.

Les moyens d'introduction des autres composants du milieu réactionnel tels qu'en particulier, l'eau, le système catalytique et des solvants éventuels ne sont pas représentés sur le schéma de la figure 1.

Un pressiostat 13 relié à la vanne d'entrée 11 du monoxyde de carbone permet de contrôler la pression totale dans le réacteur 1.

La température dans le réacteur 1 est mesurée par un dispositif thermométrique 14 et peut être modifiée en faisant circuler une fraction du liquide réactionnel dans une boucle de refroidissement traversant un échangeur de chaleur 15 alimenté par un fluide de refroidissement via une vanne 30.

Le flux de matière réactionnelle ainsi prélevé au réacteur, puis refroidi et recyclé permet d'éliminer environ 20 % de la chaleur de réaction de formation de l'acide acétique.

Les évents sortent par une canalisation 16 de la partie supérieure du réacteur 1.

Le débit de gaz purgé en tête du réacteur 1 est ajusté de manière à maintenir une pression partielle de monoxyde de carbone de 10 bar dans le ciel gazeux du réacteur.

Les évents sont ensuite introduits dans un séparateur gaz-liquide 17 qui renvoie la partie liquide dans le réacteur 1 et la partie gazeuse vers une colonne de lavage 19 dans laquelle le gaz est lavé par de l'acide acétique ou du méthanol avant d'être rejeté vers l'atmosphère après passage dans une torche 18.

Dans l'installation représentée sur la figure 1 on a prévu également d'intercaler, sur le circuit d'évacuation des évents, avant le rejet vers l'atmosphère, un débitmètre 20, un analyseur 21 permettant d'analyser les gaz émis et un module de calcul 21' connecté au débitmètre 20, à l'analyseur 21 et au débitmètre 9. L'analyseur 21 peut être tout dispositif permettant de mesurer la teneur des gaz de purge, en particulier, en hydrogène et/ou en CO₂, de façon continue ou de façon séquentielle.

Cette analyse, couplée à la mesure du débit total des évents de l'atelier permet de déterminer les débits d'hydrogène et de gaz carbonique générés par les réactions secondaires dans le réacteur de carbonylation 1.

On a ainsi accès aux sélectivités en rapportant, dans le module de calcul 21', ces débits partiels au débit total de monoxyde de carbone entrant dans le réacteur 1.

Il est possible également de vérifier par des analyses adéquates la teneur en différents éléments du milieu réactionnel, en particulier la teneur en eau et la teneur en acétate de méthyle.

On peut également prévoir de contrôler le niveau du liquide dans le réacteur. Ce contrôle peut être réalisé par tout moyen classiquement utilisé pour contrôler le niveau d'un liquide dans un réacteur, en particulier par l'intermédiaire d'une mesure de pression différentielle dans un système de jambe de référence du réacteur, représenté en 37.

Une fraction du liquide constituant le milieu réactionnel est transférée en continu par l'intermédiaire d'une canalisation 31 du réacteur 1 vers le dispositif de flash 2 dans lequel la pression est maintenue à une valeur inférieure à celle qui règne dans le réacteur 1 de façon à ce qu'il se produise une vaporisation partielle du liquide réactionnel. Le flux F6 du liquide introduit dans le dispositif de flash 2 est mesuré par l'intermédiaire d'un débitmètre 22a. La fraction non vaporisée dans la zone de flash 2 retourne vers le réacteur 1 par une canalisation 32 et son débit F2 peut être mesuré par l'intermédiaire d'un débitmètre 22b.

La fraction vaporisée dans le dispositif de flash 2 est envoyée vers la première colonne de distillation 3 par un conduit 33. Le liquide recueilli à la base de cette première colonne de distillation est renvoyé vers la zone de flash comme montré par le repère 34, alors qu'une partie de la phase liquide aqueuse de tête de cette première colonne 3 est renvoyée vers le réacteur 1 à travers un séparateur 23 de décantation des deux phases liquides obtenues avec un débit F3 qui peut être mesuré par l'intermédiaire d'un débitmètre 24, le reste de la fraction aqueuse étant renvoyé vers le haut de la première colonne de distillation 3. Par ailleurs, la phase liquide organique issue de la tête de la colonne de distillation 3 est également recyclée vers le réacteur avec un débit F4 qui peut être mesuré par l'intermédiaire d'un débitmètre 25. La fraction débarrassée des têtes et des queues de colonne issues de la colonne 3 est envoyée vers une deuxième colonne de distillation 4, par un conduit 35.

Une partie du liquide condensé en tête de cette seconde colonne 4 est recyclée avec un débit F5 vers le réacteur 1 par un conduit 36. Le débit de cette fraction peut être mesuré par l'intermédiaire d'un débitmètre 26. Le reste de la fraction de tête retourne vers la colonne 4, comme montré par le repère 39.

La fraction recueillie à la base de la deuxième colonne de distillation 4, essentiellement constituée d'acide acétique, subit une dernière étape de purification dans la colonne 5 où l'acide acétique se trouve séparé des sous-produits lourds recueillis en bas de colonne et des produits plus légers recueillis en tête de colonne et recyclés partiellement vers la colonne 4.

Lors du fonctionnement de l'installation, il arrive que le débit d'alimentation en monoxyde de carbone subit des fluctuations liées aux équipements de production situés en amont.

Le débit de méthanol est ajusté au débit de monoxyde de carbone de façon quasi-stoechiométrique.

La sélectivité CO₂ est ajustée à une valeur inférieure ou égale à 0.01
- par une concentration adéquate en rhodium dans le milieu réactionnel pour obtenir une activité catalytique suffisante,
- par l'ajustement de la concentration en promoteur iodé : l'iodure de méthyle ; il existe un stockage tampon d'iodure de méthyle dans le décanteur en tête de la première colonne de distillation. Augmenter ou diminuer ce volume tampon permet d'ajuster la teneur en iodure de méthyle dans le réacteur, et donc l'activité du système catalytique,
- par ajustement de la température du réacteur dans une plage déterminée : 175 °C à 190 °C.

De manière à sortir des états transitoires générateurs de marches incontrôlées, on cherche à trouver un régime stable qui permette de maintenir dans le réacteur les concentrations des différents constituants à des valeurs fixées et de bien contrôler les flux de retour sur le réacteur ; pour ce faire, on vise donc à maintenir la température du réacteur de manière à maximiser la production d'acide acétique selon le débit de monoxyde de carbone entrant dans le réacteur.

Le débit de liquide réactionnel vers le flash est ensuite réglé pour éliminer les calories restantes de la réaction.

Avec une conduite conventionnelle des installations, la perte de monoxyde de carbone, dit CO en excès, s'établissait à 2 % en volume, par rapport à l'ensemble du CO alimentaire.

On cherchait alors à atténuer les plus grosses fluctuations de débit de monoxyde de carbone en faisant varier par paliers la température du réacteur de manière à maximiser la production d'acide acétique et à ne pas entrer dans des régimes transitoires, sources de marches incontrôlées.

L'objectif était alors de donner une valeur fixe à la température dans le but de stabiliser les concentrations des différentes espèces dans le réacteur, ainsi que les flux.

### Exemple 2

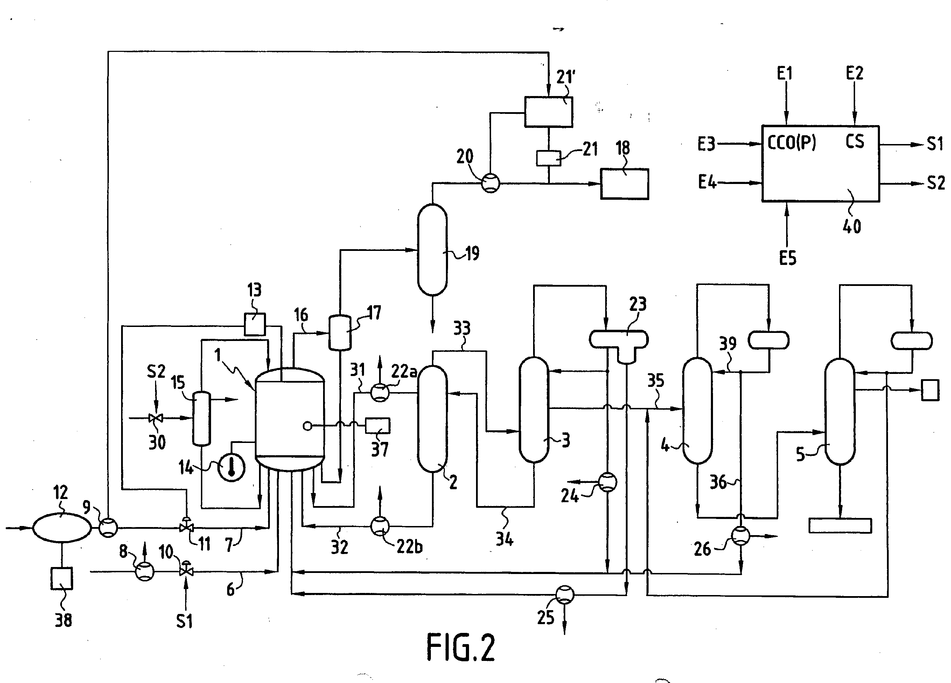

On illustre dans cet exemple la mise en oeuvre du procédé selon l'invention dans le cas d'une fabrication d'acide acétique réalisée dans les conditions opératoires de l'exemple 1 dans une installation représentée schématiquement sur la figure 2.

L'installation représentée sur la figure 2 correspond à un perfectionnement de l'installation représentée sur la figure 1, incorporant un régulateur multivariable prédictif 40.

Le régulateur 40 est programmé pour réguler des variables objectifs autour de valeurs de consigne, en agissant sur des variables d'action.

Dans cet exemple 2, les variables objectifs que l'on cherche à réguler sont le débit d'entrée du monoxyde de carbone dans le réacteur 1 et la sélectivité en CO₂. Les variables d'action sont la température à l'intérieur du réacteur 1 et le débit d'entrée du méthanol dans ce réacteur.

Plus particulièrement, le régulateur 40 reçoit :
- à une première entrée E1, le débit d'entrée du monoxyde de carbone dans le réacteur 1, tel que mesuré par le débitmètre 9 ;
- à une seconde entrée E2, la sélectivité en CO₂ de la réaction, telle que calculée par le module de calcul 21' en fonction des résultats d'analyse obtenus par l'analyseur 21, de la valeur de débit mesurée par le débitmètre 20 et de la mesure du débit d'entrée de CO donnée par le débitmètre 9 ;
- à une troisième entrée E3, le débit d'entrée du méthanol dans le réacteur 1, tel que mesuré par le débitmètre 8 ; et
- à une quatrième entrée E4, la température à l'intérieur du réacteur 1, telle que mesurée par le dispositif thermométrique 14.

Pour des raisons de clarté de la figure, les connections entre le régulateur et le reste de l'installation n'ont pas été représentées sur la figure 2.

A un instant donné, le régulateur 40 compare les variables objectifs (débit de CO et sélectivité) à des valeurs de consigne CCO et CS, et produit en sortie des signaux de régulation S1 et S2 qui dépendent de :
- l'écart entre chacune des variables objectifs et sa valeur de consigne,
- la mesure du débit d'entrée de méthanol et la mesure de la température dans le réacteur 1 et
- les relations entre les variables d'action et les variables objectifs enregistrées dans le régulateur lors de la programmation du régulateur.

Les signaux de régulation S1 et S2 sont utilisés pour commander, respectivement, la vanne 10 de commande du débit d'entrée du méthanol dans le réacteur 1 et la vanne 30 de commande du débit d'entrée du fluide de refroidissement dans l'échangeur thermique 15, de façon que le débit de monoxyde de carbone et la sélectivité en CO₂ se rapprochent de leurs valeurs de consigne CCO et CS.

La valeur de consigne CS autour de laquelle la sélectivité en CO₂ est régulée, est une valeur fixe prédéterminée choisie de préférence inférieure ou égale à 0,01.

La valeur de consigne CCO autour de laquelle le débit de monoxyde de carbone est régulé, varie en fonction de la disponibilité en monoxyde de carbone dans le réservoir de CO 12 en amont du réacteur 1. Cette disponibilité est appréhendée directement par une mesure de la pression P dans le réservoir-tampon 12 effectuée au moyen d'un pressiostat 38. La consigne CCO est calculée par le régulateur 40 en fonction de la pression P, qui est appliquée à une cinquième entrée E5 du régulateur, comme montré à la figure 2.

L'asservissement de la température dans le réacteur 1 et du débit d'entrée de méthanol dans ce même réacteur au débit de monoxyde de carbone et à la sélectivité en CO₂, tel qu'il est décrit ci-dessus, permet d'une part de diminuer le flux de monoxyde de carbone en excès, c'est-à-dire les pertes en monoxyde de carbone , et, d'autre part, de maintenir la sélectivité en CO₂ à une valeur prédéterminée, avec pour conséquence un maintien de la concentration en acétate de méthyle.

La mise en oeuvre du procédé selon l'invention dans cet exemple 2 est maintenant décrite.

Le régulateur choisi en tant que régulateur 40 est le dispositif IDCOM-HIECON, qui est un régulateur multivariable prédictif par modèle boîte noire destiné aux procédés de fabrication en continu. Il a été développé par la société ADERSA.

Ce régulateur a été préalablement programmé de la manière suivante :

Un modèle mathématique de régulation a été choisi parmi une bibliothèque de modèles donnés connus du régulateur à l'aide d'essais pré-enregistrés.

Une phase expérimentale a ensuite été mise en oeuvre, sous la forme d'essais de sollicitation du processus, sans régulation, qui se sont déroulés pendant deux jours.

Les signaux recueillis sur le site industriel à une période de 5 secondes ont été filtrés par un filtre anti-repliement et ont été sauvegardés à la période de 2 minutes pour l'identification.

Dans un premier temps, après une période de stabilisation de deux heures, la température du réacteur 1 a été incrémentée rapidement de 0,5°C ; une fois l'incrémentation de la température terminée, on a suivi l'évolution des paramètres jusqu'à obtenir un palier sur la sélectivité CO₂ et le débit d'entrée de monoxyde de carbone (durée d'environ deux heures).

La température du réacteur a de nouveau été augmentée rapidement de 0,5°C et le suivi des paramètres a été réalisé (durée environ deux heures).

Puis la température a été baissée deux fois de 0,5°C avec un suivi des paramètres.

Les mêmes opérations ont été réalisées le lendemain en baissant le débit de méthanol deux fois de 0,5 T/h, puis en l'augmentant deux fois d'un incrément de 0,5 T/h.

Le suivi des paramètres a plus particulièrement consisté à mesurer continûment les valeurs des variables d'action (température dans le réacteur 1 et débit de méthanol à l'entrée de ce même réacteur) et les valeurs de variables objectifs (débit de monoxyde de carbone à l'entrée du réacteur 1 et sélectivité en CO₂).

Le régulateur a été mis au point à l'aide du logiciel HIECON fourni par le fabricant. Les valeurs mesurées des différents paramètres ont été fournies au régulateur. Le logiciel a ensuite optimisé les paramètres de gain et de retard à appliquer aux signaux de sortie S1 et S2.

Après programmation du régulateur, celui-ci a été mis en service comme montré à la figure 2, et le procédé a été mis en oeuvre dans les mêmes conditions que dans l'exemple 1 précédemment décrit.

On a alors constaté de façon surprenante et inattendue que, grâce au régulateur 40, la sélectivité en CO₂ pouvait être maintenue dans un faible domaine de variations, tout en faisant varier la température de la masse réactionnelle et le débit d'entrée du méthanol pour pouvoir maximiser la consommation de monoxyde de carbone et donc maximiser la production d'acide acétique.

La mise en place du régulateur a permis d'abaisser à 0,5 % le débit de CO en excès par rapport au débit total de CO alimentaire, représentant ainsi un gain de productivité de 1,5% d'acide acétique sur une période représentative de 5 mois. Cela a ainsi permis d'augmenter la production d'acide acétique de plus de 2 000 tonnes, sur cette période de 5 mois, par rapport à une même quantité de CO disponible.

L'utilisation, selon l'invention, d'un régulateur multivariable à commande prédictive dans un procédé tel que décrit dans l'exemple comparatif 1 a permis de satisfaire les différentes contraintes parfois antagonistes : en effet, selon l'art antérieur, on cherchait à maintenir une température la plus constante possible pour stabiliser les autres paramètres, mais le débit de monoxyde de carbone présentait des fluctuations.

### Exemple 3

On reprend dans cet exemple l'installation industrielle et le dispositif mis en place dans l'exemple 2.

Toutefois, le régulateur 40 est utilisé ici pour asservir en outre les flux de recyclage vers le réacteur 1 au niveau du liquide dans ce réacteur.

Les principaux flux liquides d'entrée dans le réacteur 1 sont :
- le débit d'alimentation en méthanol - F1 -
- les flux de recyclage des zones II et III vers le réacteur, à savoir :
   - le recyclage de la fraction liquide du flash augmentée du débit de liquide provenant de la base de la première colonne de distillation - F2 -
   - le recyclage d'une partie de la phase liquide aqueuse de la tête de la première colonne de distillation - F3 -
   - le recyclage de la phase liquide organique de la tête de la première colonne de distillation - F4 -
   - le recyclage d'une partie du liquide condensé en tête de la seconde colonne de distillation - F5 -

Le principal flux liquide de sortie du réacteur est constitué par le débit allant du réacteur vers le flash - F6 -.

Le flux F1 étant déjà incorporé au régulateur multivariable, il suffit d'ajouter, comme variables d'action, les flux F2, F3, F4, F5 et F6 mesurés respectivement par les débitmètres 22b, 24, 25, 26 et 22a, et comme variable objectif, le niveau de liquide dans le réacteur 1, mesuré par le dispositif 37.

Le nombre de variables objectifs est donc de trois :
- le débit de CO consommé,
- la sélectivité CO₂,
- le niveau du liquide dans le réacteur,
et le nombre de variables d'action est de sept :
- le débit de méthanol F1,
- la température du réacteur,
- les débits F2, F3, F4, F5, F6.

Cependant, le nouvel objectif de régulation, relatif au niveau de liquide dans le réacteur, peut être obtenu de différentes manières.

Par exemple, il est possible de ne retenir, dans les variables d'action de débit, que les débits F2, F4, F5 et F6 (c'est-à-dire d'omettre le débit F3), en ajoutant dans le régulateur, en contrepartie, une relation fixée entre les débits F4 et F3 comme objectif secondaire.

Afin de réduire encore le nombre de variables d'action, il est possible également de se fixer des objectifs secondaires complémentaires, tels que le maintien de la valeur du rapport F2/F1 et le maintien de la valeur du rapport F6/F1.

On peut ainsi gérer de façon automatique les principaux flux liquides de l'installation de production d'acide acétique tout en maximisant la production d'acide acétique en utilisant au mieux le monoxyde de carbone disponible.

Dans cet exemple 3, les essais préalables à la programmation du régulateur 40 ont été réalisés de la façon suivante :

Les relations entre les différentes variables ont été construites en effectuant pour chaque couple variable d'action/variable objectif, ici en particulier entre d'une part, les différents débits, et d'autre part, le niveau de liquide dans le réacteur, plusieurs variations sur la variable d'action et en observant leur influence sur la variable objectif, tout en maintenant constants les autres paramètres.

Les signaux recueillis sur le site industriel à une période de 5 secondes ont été filtrés par un filtre anti-repliement et ont été sauvegardés à la période de 2 minutes pour l'identification.

Les essais se sont déroulés pendant plusieurs jours, avec modification d'un seul des débits (F1, F2, F4, F5, F6) par incréments sur une période d'un jour ; le débit F3 ayant été fixé par consigne et en proportion directe du débit d'alimentation de méthanol.

Les incréments de chaque paramètre ont été choisis selon l'ampleur des perturbations entraînées sur l'ensemble de l'atelier.

Ainsi, les incréments positifs ou négatifs ont été de 0,2 T/h pour F1, 2 T/h pour F2, 0,5 T/h pour F4 et F5 et de 15 T/h pour F6.

Le logiciel HIECON a ensuite été utilisé pour optimiser les paramètres de régulation.

Puis le régulateur a été mis en service dans l'installation.

### Exemple 4

Dans cet exemple, la carbonylation du méthanol est conduite dans une installation pilote en continu, qui reprend le principe de fonctionnement de l'atelier industriel, avec un réacteur agité, un flash, le recyclage de la phase liquide (contenant l'espèce catalytique) du séparateur flash vers le réacteur.

La distillation n'est pas effectuée et le recyclage des flux de la zone III est simulé par introduction de réactifs dits neufs (mélange d'acide acétique, d'acétate de méthyle, d'iodure de méthyle et d'eau) ; le catalyseur perdu par entraînement dans la phase vapeur du séparateur flash est ré-introduit périodiquement sous forme de catalyseur neuf dans le réacteur.

La composition du milieu réactionnel est la suivante :
- Eau : 7%
- Iodure de méthyle : 10 %
- Acétate de méthyle : 15%
- Iridium : 1250 ppm
- Acide acétique : complément à 100%

La pression totale est de 35 bar, la température du réacteur est de 190 °C.

La pression partielle en monoxyde de carbone est de 23 bar.

La vitesse de carbonylation est de 15,5 moles/(h.l) dans ces conditions.

La sélectivité CO₂ est de 0.01, la sélectivité hydrogène vaut 0.001 et la sélectivité méthane 0.009.

Dans cet exemple d'une installation pilote, l'objectif principal n'est pas une optimisation de l'utilisation du monoxyde de carbone disponible, mais réside dans l'obtention de conditions de réaction et de fonctionnement suffisamment stables pour valider et optimiser l'activité du milieu catalytique.

Les paramètres de la conduite de la catalyse au rhodium de l'exemple 2 ne peuvent plus être utilisés : en effet la variation de la sélectivité CO₂ est peu sensible dans la plage de fonctionnement retenue, et donc ne permet pas de contrôler la teneur en acétate de méthyle.

Par ailleurs la chimie à basse teneur en eau est très sensible à la teneur en eau qui est un paramètre direct de la cinétique de la réaction de production d'acide acétique.

Cela impose donc un suivi continu de cette teneur en eau dans le réacteur :
- soit par mesure directe
- soit par calcul tenant compte des concentrations en eau des différents flux de recycle vers le réacteur.

Un analyseur par spectrométrie proche infra-rouge a permis de doser in-situ les teneurs en eau, en acétate de méthyle, en iodure de méthyle et en acide acétique dans le milieu réactionnel, avec des temps de réponse inférieurs à la minute.

L'analyseur mis en place est le modèle 5000 de la société NIRSystems.

Il a ensuite été étalonné de la manière suivante :
- l'analyseur a été placé en acquisition automatique (sauvegarde des spectres)
- le prélèvement d'un échantillon est effectué à chaque point de fonctionnement différent
- l'échantillon est dosé par chromatographie en phase gazeuse (méthode de référence)
- le spectre correspondant au temps de prélèvement est sélectionné
- la base de données spectres - concentrations est enrichie.

Un modèle mathématique est alors créé pour déterminer en temps réel, les concentrations des trois constituants principaux sélectionnés : eau, acétate de méthyle et iodure de méthyle.

Les erreurs de prédiction ont été ensuite déterminées :
- eau : 0.2% pour une gamme de 0,5 % à 21 %
- acétate de méthyle : 0,4% pour une gamme entre 0,5 % et 24 %
- iodure de méthyle : 0,7% pour une gamme entre 2 % et 14%

Le régulateur est utilisé dans cet exemple 4 pour réguler deux variables objectifs :
- le débit de CO consommé,
- la teneur en eau dans le milieu réactionnel,
en agissant sur deux variables d'action :
- la température du milieu réactionnel dans le réacteur de carbonylation,
- le débit de méthanol alimentaire.

La mise en place de ce régulateur a permis de maintenir de façon automatique, sans intervention humaine, à des valeurs prédéterminées :
- le débit de CO consommé,
- la teneur en eau,
pendant des cycles entiers de plusieurs heures d'essais de systèmes catalytiques à basse teneur en eau.

Les essais préalables à la programmation du régulateur ont consisté, de manière générale, à effectuer pour chaque couple variable d'action/variable objectif, plusieurs variations sur la variable d'action et à observer leur influence sur la variable objectif, tout en maintenant constants les autres paramètres.

Les signaux recueillis sur le site industriel à une période de 5 secondes ont été filtrés par un filtre anti-repliement et ont été sauvegardés à la période de 2 minutes pour l'identification.

Le logiciel HIECON a permis ensuite d'adapter le régulateur au problème.

## Revendications

1. Procédé de préparation en continu d'acide acétique et/ou d'acétate de méthyle par carbonylation par du monoxyde de carbone, du méthanol, ou d'un dérivé carbonylable du méthanol, en phase liquide, en présence d'eau et d'un système catalytique homogène, ledit procédé de préparation étant mis en oeuvre dans une installation industrielle comprenant :
• Une zone I dite zone de réaction, comprenant un réacteur dans lequel est réalisée ladite réaction de carbonylation du méthanol, en phase liquide, à une température de 150 à 250 °C, sous une pression de 5.10⁵ à 200.10⁵ Pa et avec élimination sous forme d'évents d'une partie du ciel gazeux au dessus du niveau liquide du milieu réactionnel dans ledit réacteur,
• Une zone II dite zone de vaporisation ou zone de flash où le liquide issu du milieu réactionnel de la zone I est partiellement vaporisé à une pression inférieure à celle de la zone I, la fraction liquide issue de cette vaporisation partielle étant recyclée vers le réacteur,
• Une zone III dite zone de purification par distillation de la fraction vaporisée issue dudit flash de la zone II, sur une ou plusieurs colonnes de distillation, à la sortie de laquelle/desquelles on récupère l'acide acétique et/ou l'acétate de méthyle, les autres constituants de ladite fraction vaporisée étant au moins partiellement recyclés vers ledit réacteur,
**caractérisé en ce que**, en vue d'améliorer le contrôle de la production d'acide acétique et/ou d'acétate de méthyle, on asservit la température du réacteur et le débit d'entrée du méthanol ou du dérivé carbonylable dans ledit réacteur au débit d'entrée de monoxyde de carbone et à au moins un des paramètres définissant la composition du milieu réactionnel et/ou des évents.

2. Procédé selon la revendication 1, **caractérisé en ce que** l'asservissement est réalisé par l'intermédiaire d'un régulateur multivariable prédictif.

3. Procédé selon la revendication 1 ou 2, **caractérisé en ce qu'**il est mis en oeuvre en maintenant la concentration en eau dans le milieu réactionnel à une valeur supérieure à 14 % en poids.

4. Procédé selon la revendication 3, **caractérisé en ce que**, la température du réacteur et le débit d'entrée du méthanol sont asservis au débit d'entrée de monoxyde de carbone et la sélectivité en H₂ ou en C0₂ maintenue à une valeur inférieure ou égale à 0,01 et/ou à la concentration en acétate de méthyle maintenue à une valeur inférieure à 5 %, de préférence inférieure à 2 %, en poids par rapport au poids du milieu réactionnel.

5. Procédé selon l'une des revendications 1 ou 2, **caractérisé en ce qu'**il est mis en oeuvre en maintenant la concentration en eau dans le milieu réactionnel à une valeur inférieure à 14% en poids par rapport au poids dudit milieu réactionnel.

6. Procédé selon la revendication 5, **caractérisé en ce que** la température du réacteur et le débit d'entrée du méthanol ou du dérivé carbonylable dans ledit réacteur sont asservis au débit d'entrée de monoxyde de carbone et à la concentration en eau dans le milieu réactionnel, ladite concentration étant maintenue à une valeur de consigne prédéterminée.

7. Procédé selon l'une des revendications 1 à 6, **caractérisé en ce que** le débit de liquide du réacteur vers la zone II de flash et les débits de liquide de recyclage des zones II et III dans ledit réacteur sont, en outre, asservis au niveau du liquide dans ledit réacteur, de façon à ce que ledit niveau reste fixé à une valeur prédéterminée.

8. Procédé selon l'une des revendications 1 à 7, **caractérisé en ce que** l'on contrôle et régule la concentration en eau dans le milieu réactionnel.

9. Procédé selon l'une des revendications 1 à 8, **caractérisé en ce que** l'on amortit les variations du débit d'entrée du monoxyde de carbone par l'intermédiaire d'un réservoir tampon placé en amont dudit réacteur.

10. Procédé selon la revendication 9, **caractérisé en ce que** l'on fait dépendre une valeur de consigne du débit de monoxyde de carbone de la pression à l'intérieur dudit réservoir tampon.

11. Procédé selon l'une des revendications 1 à 10, **caractérisé en ce que** l'on amortit les fluctuations de débit du monoxyde de carbone entrant dans le réacteur en rejetant au moins une partie du débit excédentaire à l'atmosphère.

12. Procédé selon l'une des revendications 1 à 11, **caractérisé en ce qu'**une partie des calories de la réaction de production d'acide acétique est éliminée ou récupérée.

13. Procédé selon l'une des revendications 1 à 12, **caractérisé en ce que** l'on mesure les concentrations en eau et en acétate de méthyle par l'intermédiaire d'un analyseur opérant dans le domaine du proche infra-rouge.

14. Procédé selon l'une des revendications 1 à 13, **caractérisé en ce que** le système catalytique mis en oeuvre dans le procédé de préparation de l'acide acétique et/ou de l'acétate de méthyle comprend au moins un métal du groupe VIII, en particulier du rhodium, de l'iridium, ou du platine.

15. Procédé selon la revendication 14, **caractérisé en ce que** ledit système catalytique comprend en outre au moins un co-catalyseur.

16. Procédé selon la revendication 15, **caractérisé en ce que** ledit co-catalyseur est l'iodure de méthyle.

17. Procédé selon la revendication 16, **caractérisé en ce que** l'on mesure les concentrations en eau et en acétate de méthyle et/ou en iodure de méthyle par l'intermédiaire d'un analyseur opérant dans le domaine du proche infra-rouge.

18. Procédé selon l'une des revendications 1 à 17, **caractérisé en ce qu'**il est appliqué à une production d'acide acétique et/ou d'acétate de méthyle par carbonylation de méthanol.

## Claims

1. A method for the continuous preparation of acetic acid and/or methyl acetate by the carbonylation with carbon monoxide of methanol or a carbonylatable derivative of methanol, in the liquid phase, in the presence of water and a homogeneous catalyst system, said preparation process being carried out in an industrial installation comprising:
• a zone I, called a reaction zone, comprising a reactor in which said methanol carbonylation reaction is carried out in the liquid phase at a temperature of 150 to 250°C, under a pressure of 5.10⁵ to 200.10⁵ Pa and with the venting of part of the gaseous canopy above the liquid level of the reaction medium in said reactor;
• a zone II, called a vaporization zone or flash zone, in which the liquid originating from the reaction medium in zone I is partially vaporized at a pressure below that of zone I, the liquid fraction originating from this partial vaporization being recycled into the reactor; and
• a zone III, called a purification zone, in which the vaporized fraction originating from said flash zone II is distilled on one or more distillation columns, at the outlet of which the acetic acid and/or methyl acetate are recovered, the other constituents of said vaporized fraction being at least partially recycled into said reactor,
wherein, with the view of improving the control of the production of acetic acid and/or of methyl acetate, the reactor temperature and the feed rate of the methanol or carbonylatable derivative in said reactor by the carbon monoxide feed rate and by at least one of the parameters defining the composition of the reaction medium and/or the vents.

2. The process according to claim 1 wherein the control is effected via a multivariable predictive controller.

3. The process according to claim 1 or 2, wherein it is carried out, maintaining the water concentration in the reaction medium at a value greater than or equal to 14 % by weight.

4. The process according to claim 3 wherein the reactor temperature and the methanol feed rate are controlled by the carbon monoxide feed rate and the H₂ or CO₂ selectivity maintained at a value less than or equal to 0.01, and/or the methyl acetate concentration maintained at a value of less than 5% by weight, preferably of less than 2% by weight, based on the weight of the reaction medium.

5. The process according to claim 1 or 2, wherein it is carried out, maintaining the water concentration in the reaction medium at a value below 14% by weight, based on the weight of said reaction medium.

6. The process according to claim 5 wherein the reactor temperature and the flow rate of methanol or carbonylatable derivative feeding said reactor are controlled by the carbon monoxide feed rate and the water concentration in the reaction medium, said concentration being maintained at a predetermined set value.

7. The process according to one of claims 1 to 6 wherein the flow rate of liquid from the reactor into flash zone II and the flow rates of recycle liquid from zones II and III into said reactor are also controlled by the liquid level in said reactor so that said level remains fixed at a predetermined value.

8. The process according to one of claims 1 to 7 wherein the water concentration in the reaction medium is controlled and regulated.

9. The process according to one of claims 1 to 8 wherein the variations in the carbon monoxide feed rate are damped via a buffer reservoir placed upstream of said reactor.

10. The process according to claim 9 wherein a set value of the carbon monoxide flow rate is made to depend on the pressure inside said buffer reservoir.

11. The process according to one of claims 1 to 10 wherein the fluctuations in the flow rate of carbon monoxide feeding the reactor are damped by discharging at least a part of the excess flow into the atmosphere.

12. The process according to one of claims 1 to 11 wherein part of the heat of the acetic acid production reaction is removed or recovered.

13. The process according to one of claims 1 to 12 wherein the concentrations of water and methyl acetate are measured by means of an analyzer operating in the near infrared region.

14. The process according to one of claims 1 to 13 wherein the catalyst system used in the process for the preparation of acetic acid and/or methyl acetate comprises at least one group VIII metal, particularly rhodium, iridium or platinum.

15. The process according to claim 14 wherein said catalyst system also comprises at least a co-catalyst.

16. The process according to claim 15, wherein said co-catalyst is methyl iodide.

17. The process according to claim 16 wherein the concentrations of water and methyl acetate and/or methyl iodide are measured by means of an analyzer operating in the near infrared region.

18. The process according to one of claims 1 to 17 which is applied to the production of acetic acid and/or methyl acetate by the carbonylation of methanol.

## Patentansprüche

1. Verfahren zur kontinuierlichen Herstellung von Essigsäure und/oder Methylacetat durch Carbonylierung durch Kohlenmonoxyd von Methanol oder einem carbonylierbaren Derivat von Methanol in Flüssigphase, in Gegenwart von Wasser und einem homogenen, katalytischen System, wobei das Herstellungsverfahren in einer industriellen Anlage ausgeführt wird, welche umfaßt:
- eine Zone I, genannt Reaktionszone, umfassend einen Reaktor, in welchem eine Carbonylierungsreaktion des Methanols in Flüssigphase ausgeführt wird, bei einer Temperatur von 150 bis 250°C unter einem Druck von 5 x 10⁵ bis 200 x 10⁵ Pa und mit Entfernung von einem gasförmigen Kopfteil über dem Flüssigkeitspegel des Reaktionsmediums in dem Reaktor in Form von Abzugsöffnungen,
- eine Zone II, genannt Verdampfungszone oder Flashzone, wo die Flüssigkeit aus dem Reaktionsmedium der Zone I partiell bei einem Druck unterhalb von jenem der Zone I verdampft wird, wobei die Flüssigfraktion aus dieser partiellen Verdampfung zum Reaktor rezykliert wird,
- eine Zone III, genannt Reinigungszone durch Destillation der verdampften Fraktion aus der Flashzone II auf einer oder mehreren Destillationskolonnen, an deren Ausgang Essigsäure und/oder Methylacetat gewonnen wird, wobei die anderen Bestandteile der verdampften Fraktion wenigstens teilweise zu dem Reaktor rezykliert werden,
**dadurch kennzeichnet, daß** im Hinblick auf die Verbesserung der Regelung der Produktion von Essigsäure und/oder Methylacetat die Temperatur des Reaktors und der Eingangsdurchsatz des Methanols oder des carbonylierbaren Derivats in den Reaktor dem Eingangsdurchsatz von Kohlenmonoxid und wenigstens einem der Parameter unterworfen werden, die die Zusammensetzung des Reaktionsmediums definieren und/oder die Abzugsöffnungen.

2. Verfahren nach Anspruch 1, **dadurch gekennzeichnet, daß** die Unterwerfung mittels einer mehrfach variablen Vorhersagesteuerung durchgeführt wird.

3. Verfahren nach Anspruch 1 oder 2, **dadurch gekennzeichnet, daß** es ausgeführt wird, in dem die Wasserkonzentration im Reaktionsmedium bei einem Wert oberhalb von 14 Gew.-% gehalten wird.

4. Verfahren nach Anspruch 3, **dadurch gekennzeichnet, daß** die Temperatur des Reaktors und der Eingangsdurchsatz des Methanols dem Eingangsdurchsatz von Kohlenmonoxid unterworfen werden und die Selektivität an H₂ oder CO₂ bei einem Wert unterhalb oder gleich 0,01 gehalten wird und/oder die Konzentration an Methylacetat bei einem Wert unter 5 Gew.-%, vorzugsweise unter 2 Gew.-% im Verhältnis zum Reaktionsmedium gehalten wird.

5. Verfahren nach Anspruch 1 oder 2, **dadurch gekennzeichnet, daß** es unter Aufrechterhaltung der Wasserkonzentration in dem Reaktionsmedium bei einem Wert unter 14 Gew.-% im Verhältnis zum Gewicht des Reaktionsmediums ausgeführt wird.

6. Verfahren nach Anspruch 5, **dadurch gekennzeichnet, daß** die Temperatur des Reaktors und der Eingangsdurchsatz von Methanol oder carbonylierbaren Derivat in dem Reaktor dem Eingangsdurchsatz von Kohlenmonoxid und der Wasserkonzentration im Reaktionsmedium unterworfen werden, wobei die Konzentration auf einem vorbestimmten Sollwert gehalten wird.

7. Verfahren nach Anspruch 1 bis 6, **dadurch gekennzeichnet, daß** der Flüssigdurchsatz des Reaktors zur Flashzone II und die Flüssigrezyklierungsdurchsätze der Zonen II und III in dem Reaktor im übrigen dem Niveau der Flüssigkeit in dem Reaktor derart unterworfen werden, daß der Pegel auf einem vorbestimmten Wert festgelegt bleibt.

8. Verfahren nach einem der Ansprüche 1 bis 7, **dadurch gekennzeichnet, daß** man die Wasserkonzentration im Reaktionsmedium kontrolliert und reguliert.

9. Verfahren nach einem der Ansprüche 1 bis 8, **dadurch gekennzeichnet, daß** man die Variationen des Eingangsdurchsatzes von Kohlenmonoxid mittels eines Puffertanks dämpft, der dem Reaktor vorgelagert ist.

10. Verfahren nach Anspruch 9, **dadurch gekennzeichnet, daß** man einen Sollwert des Durchsatzes von Kohlenmonoxid von dem Druck in dem Puffertank abhängen läßt.

11. Verfahren nach einem der Ansprüche 1 bis 10, **dadurch gekennzeichnet, daß** man die Fluktuationen des Durchsatzes von Kohlenmonoxid, das in den Reaktor eintritt, dämpft, indem man wenigstens einen Teil des überschüssigen Durchsatzes in die Atmosphäre ausstößt.

12. Verfahren nach einem der Ansprüche 1 bis 11, **dadurch gekennzeichnet, daß** ein Teil der Kalorien der Reaktion zur Herstellung von Essigsäure beseitigt oder rückgewonnen wird.

13. Verfahren nach einem der Ansprüche 1 bis 12, **dadurch gekennzeichnet, daß** man die Konzentrationen an Wasser und an Methylacetat mit einem Analysator mißt, der im Nahinfrarotbereich arbeitet.

14. Verfahren nach einem der Ansprüche 1 bis 13, **dadurch gekennzeichnet, daß** das in dem Verfahren zur Herstellung von Essigsäure und/oder Methylacetat eingesetzte katalytische System wenigstens ein Metall der Gruppe VIII umfaßt, insbesondere Rhodium, Iridium oder Platin.

15. Verfahren nach Anspruch 14, **dadurch gekennzeichnet, daß** das katalytische System im übrigen wenigstens einen Co-Katalysator umfaßt.

16. Verfahren nach Anspruch 15, **dadurch gekennzeichnet, daß** der Co-Katalysator Methyliodid ist.

17. Verfahren nach Anspruch 16, **dadurch gekennzeichnet, daß** man die Konzentrationen an Wasser und Methylacetat und/oder Methyliodid mittels eines Analysators mißt, der im Nahinfrarotbereich arbeitet.

18. Verfahren nach einem der Ansprüche 1 bis 17, **dadurch gekennzeichnet, daß** es auf eine Herstellung von Essigsäure und/oder Methylacetat durch Carbonylierung von Methanol angewandt wird.
